Europäisches Patentamt

European Patent Office

Office européen des brevets

(19)

(11) **EP 0 837 881 B1**

(12) **FASCICULE DE BREVET EUROPEEN**

(45) Date de publication et mention
de la délivrance du brevet:
**15.09.2004 Bulletin 2004/38**

(51) Int Cl.[7]: **C07K 14/685**, A61K 47/48

(86) Numéro de dépôt international:
**PCT/FR1996/000890**

(21) Numéro de dépôt: **96922103.5**

(22) Date de dépôt: **12.06.1996**

(87) Numéro de publication internationale:
**WO 1996/041815 (27.12.1996 Gazette 1996/56)**

(54) **CONJUGUES D'alpha-MSH AVEC UN ACIDE GRAS, LEUR PROCEDE DE PREPARATION ET LEUR UTILISATION A TITRE DE MEDICAMENT**

KONJUGATE VON alpha-MSH MIT EINER FETTSÄURE, VERFAHREN ZU IHRER HERSTELLUNG UND IHRE VERWENDUNG ALS MEDIKAMENT

CONJUGATES OF alpha-MSH WITH A FATTY ACID, PREPARATION METHOD THEREFOR, AND USE THEREOF AS A DRUG

(84) Etats contractants désignés:
**AT BE CH DE DK ES FI FR GB GR IE IT LI LU MC NL PT SE**

(30) Priorité: **12.06.1995 FR 9506909**

(43) Date de publication de la demande:
**29.04.1998 Bulletin 1998/18**

(73) Titulaire: **INSTITUT EUROPEEN DE BIOLOGIE CELLULAIRE**
**F-31520 Ramonville-Saint-Agne (FR)**

(72) Inventeurs:
• **DUSSOURD D'HINTERLAND, Lucien**
**F-31400 Toulouse (FR)**
• **PINEL, Anne-Marie**
**F-34280 La Grande-Motte (FR)**

(74) Mandataire: **Warcoin, Jacques**
**Cabinet Régimbeau**
**20, rue de Chazelles**
**75847 Paris cedex 17 (FR)**

(56) Documents cités:
**EP-A- 0 389 950**     **EP-A- 0 517 211**
**WO-A-95/08564**     **FR-A- 2 691 465**

• **BIOCHEMISTRY, vol. 32, no. 45, 16 Novembre 1993, pages 12264-12272, XP000403772 ITO A S ET AL: "STRUCTURE-ACTIVITY CORRELATIONS OF MELANOTROPIC PETIDES IN MODEL LIPIDS BY TRYPTOPHAN FLUORESCENCE STUDIES"**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention a pour objet la réalisation de dérivés peptidiques homologues de l'αMSH ou Mélanotropine, obtenus par synthèse chimique, présentés sous forme de lipo-peptides.

L'αMSH et ses homologues ont fait l'objet de nombreuses publications, voire même d'essais thérapeutiques sans que cela se traduise par la réalisation d'un médicament. La raison principale est l'extrême ubiquité de l'αMSH en fonction des doses administrées et des voies d'administration.

De plus, l'absence de relation doses / effets (ce qui est le cas de nombreuses hormones peptidiques) complique sérieusement son utilisation thérapeutique. En effet, les récepteurs cellulaires de l'αMSH et de ses homologues sont du type G et la transduction intra-cellulaire s'effectue selon le cycle de l'AMP cyclique, l'activation de ces récepteurs cellulaires est sensiblement inversement proportionnelle aux doses d'hormones peptidiques; acceptées par les structures du récepteur membranaire.

La demande de brevet FR 2 691 465 divulgue des complexes peptidiques présentant une séquence d'au moins 4 acides aminés extraits de l'α-MSH conjugués à un élément polysacharridique, de formule

$$X-Glu-His-A-Arg-Trp-Gly-X$$

Ou A représente Phe ou DPhe et

X représente une séquence d'acides aminés où l'acide aminé terminal comporte éventuellement un groupe acétyle. Ces complexes possèdent une activité anti-inflammatoire, anti-allergique et/ou activatrice de la mélanogénèse.

La demande de brevet EP 389 950 divulgue des dérivés peptidiques inhibiteurs de l'α-MSH.

L'article Biochemistry, vol. 32, N° 45, 16 novembre 1993, pages 12264-12272, ITO A S et al. « Structure-activity corrélations of melanotropic peptides in model lipids by tryptophan fluorescence studies » divulgue des analogues de l'α-MSH associés à des lipides.

La demande de brevet WO95/08564 décrit des dérivés peptidiques de l'α-MSH conjugués avec l'acide thioctique ou un de ses dérivés.

Enfin, la demande de brevet EP 517 211 décrit une composition pharmaceutique comprenant une quantité efficace d'un polypeptide actif avec un promoteur d'absorption consistant en la combinaison d'un acide organique et d'un ester de sucrase d'acide gras, le poly-peptididique pouvant être l'α-MSH.

La présente invention repose sur la recherche de structures peptidiques spécifiquement orientées vers les activités anti-allergiques et anti-inflammatoires d'une part, et activatrices de la mélanogénèse d'autre part à l'exclusion de tout autre effet pharmacologique notamment au niveau du système nerveux central, comme du système nerveux périphérique.

La présente invention concerne un conjugué comportant une séquence peptidique comprenant au moins une séquence de 4 acides aminés dérivés de l'αMSH, les acides aminés étant sous forme naturelle ou non, ladite séquence étant conjuguée, chimiquement ou physiquement avec des acides notamment dérivés d'acides à activités métaboliques connues, sélectionnés parmi :

- les acides dicarboxyliques de formule générale I

$$HOOC - R1 - COOH \qquad (I)$$

dans laquelle R1 représente un radical alkylène, linéaire ou ramifié, comprenant au moins 3 atomes de carbone de préférence ayant de 3 à 10 atomes de carbone, éventuellement substitué, notamment par un ou plusieurs groupes amino ou hydroxy, et
- les acides gras α-monoinsaturés de formule générale II

$$R2 - CH = CH - COOH \qquad (II)$$

dans laquelle R2 représente un radical alkyle, linéaire ou ramifié comprenant au moins 6 atomes de carbone, de préférence de 6 à 10 atomes de carbone éventuellement substitué par un groupe amino, hydroxy ou oxo, ces acides ayant la configuration cis ou trans, de préférence trans.

La séquence de l'αMSH est la suivante :

## N-acétyl-Ser-Tyr-Ser-Met-Glu-His-Phé-Arg-Trp-Gly-Lys-Pro-Val-NH$_2$

Dans ce qui suit, lorsque l'on mentionne les séquences provenant de l'aMSH il faut entendre que les acides aminés peuvent être sous forme D, L, ou D,L ou bien sous les formes non naturelles des acides aminés correspondant à des dérivés, notamment substitués, halogénés par exemple.

Les peptides conjugués de bas poids moléculaire selon l'invention sont avantageusement liés sous forme de sels, d'esters ou d'amides, à des acides possédant des fonctions métaboliques essentielles dans l'activation du cycle tricarboaylique cellulaire tels qu'ils sont décrits précédemment.

Les acides de formules générales I ou II sont de préférence choisis parmi les diacides carboxyliques pour lesquels R1 représente un reste alkylène en C$_4$-C$_8$, substitué ou non, en particulier les acides adipiques, α-amino-adipiques et dérivés, l'acide sébacique et dérivés, les acides gras α-monoinsaturés pour lesquels R2 représente un reste alkyle, linéaire ou ramifié en C$_7$, en particulier les acides hydroxydécénoïques, et décénoïliques, sous forme des sels, esters, ou amides correspondants.

Il s'agit de préférence de l'acide adipique ou "acide hexanedioïque" (ADIP), de l'acide α-amino adipique (2-amino hexanedioïque), de l'acide sébacique ou acide décanedioïque, de l'acide trans - 10 - hydroxy - $\Delta^2$ - décenoïque ou acide trans-hydroxy - 10 - décène - 2 - oïque (AH), et de l'acide trans-oxo - 9 - décène - 2 - oïque (9 - ADO).

Parmi ces acides, figurent préférentiellement pour des applications en phase soluble dans l'eau, les acides adipiques et amino-adipiques.

Parmi les acides figurent les acides gras suivants plus particulièrement adaptés pour l'obtention de dérivés peptidiques liposolubles, l'acide sébacique et ses dérivés, l'acide oxo-décéné-oïque, et l'acide trans-hydroxy-décénoïque, définis précédemment, et dont les activités biologiques sont liées à leurs propriétés, anti-androgène, anti-séborrhéique et lipolytiques (Madéa et coll., Nippon, Hifuka - 98 (4) p. 469 - 1988).

Plus spécifiquement, la présente invention concerne des peptides activateurs de la mélanogénèse, anti-allergique et anti-inflammatoire.

La séquence de 4 acides aminés provenant de l' αMSH, des conjugués selon l'invention, comprend au moins l'une des séquences peptidiques suivantes :

$$- Glu - His - Phé -$$

$$- His - Phé - Arg -$$

$$- Glu - His - Phé - Arg -$$

dans laquelle Phé représente la phénylalanine ou un dérivé halogène, de la phénylalanine, notamment D-homo-Phé et parafluoro-Phé les acides aminés pouvant être sous forme D, L, ou D,L.

Les conjugués peptidiques selon l'invention répondent à la formule générale III :

$$A - X - Glu - His - Phé - Y \quad (III)$$

dans laquelle

A est un acide de formules générales I ou II telles que définies ci-dessus, en particulier l'acide adipique, ou l'acide α-amino adipique, l'acide sébacique, l'acide trans-oxo-9-décéne-2-oïque ou l'acide trans-hydroxy-10-décéne-2-oïque,

X représente un groupe 5-Me.Norleucine (Me.Nle), 2-N-Me-Norceuline (N-Me.Nle), OH ou NH$_2$,

Y représente un reste Arg - Z ou Z dans lequel Z est Trp - Gly - OH, Trp - OH, OH, Trp - Gly - NH$_2$, Trp - NH$_2$, ou NH$_2$,

Phé représente un reste D-Homo-Phé ou para-fluoro-Phé,

les acides aminés étant sous forme D, L ou D,L.

La présente invention concerne particulièrement les dérivés peptidiques suivants :

1.      A - Me.Nle - Glu - His - D-homo-Phé - Arg - Trp - Gly - NH$_2$

2.      A - Me.Nle - Glu - His - D-homo-Phé - Arg - Trp - NH$_2$

3        A - Me.Nle - Glu - His - D-homo-Phé - Arg - NH₂

4        A - Me.Nle - Glu - His - D-homo-Phé - NH₂

5        A - Me.Nle - Glu - His - para-fluoro-Phé - Arg - Trp - Gly - NH₂

6        A - Me.Nle - Glu - His - para-fluoro-Phé - Arg - Trp - NH₂

7        A - Me.Nle - Glu - His - para-fluoro-Phé - Arg - NH₂

8        A - Me.Nle - Glu - His - para-fluoro-Phé - NH₂

9        A - N - Me.Nle - Glu - His - D-homo-Phé - Arg - Trp - Gly - NH₂

10        A - N - Me.Nle - Glu - His - D-homo-Phé - Arg - Trp - NH₂

11        A - N - Me.Nle - Glu - His - D-homo-Phé - Arg - NH₂

12        A - N - Me.Nle - Glu - His - D-homo-Phé - NH₂

13        A - Me.Nle - Glu - His - D-homo-Phé - Arg - Trp - Gly - OH

14        A - Me.Nle - Glu - His - D-homo-Phé - Arg - Trp - OH

15        A - Me.Nle - Glu - His - D-homo-Phé - Arg - OH

16        A - N - Me.Nle - Glu - His - D-homo-Phé - Arg - Trp - Gly - OH

17        A - N - Me.Nle - Glu - His - D-homo-Phé - Arg - Trp - OH

18        A - Me.Nle - Glu - His - para-fluoro-Phé - Arg - Trp - Gly - OH

19        A - Me.Nle - Glu - His - para-fluoro-Phé - Arg - Trp - OH

20        A - Me.Nle - Glu - His - para-fluoro-Phé - Arg - OH

21        A - N - Me.Nle - Glu - His - para-fluoro-Phé - Arg - Trp - Gly - OH

22    A - N - Me.Nle - Glu - His - para-fluoro-Phé - Arg - Trp - OH

A étant défini ci-dessus, ainsi que les dérivés de ces molécules sous forme de sels d'esters ou d'amides.

Dans les conjugués 1 à 22 ci-dessus, la position de l'acide A correspond au dérivé ester ou amide, la fraction acide carboxylique de l'acide assurant la liaison.

Les séquences d'amino-acides mentionnées précédemment peuvent être des séquences d'amino-acides naturels ou des séquences d'amino-acides non naturels. De même dans certains cas il est possible que certains de ces amino-acides comportent des fonctions par exemple des glycosylations. Il doit être entendu que la présente invention concerne également l'ensemble de ces formes comprises par la présente description.

La présente invention concerne également des médicaments contenant un ou plusieurs des conjugués selon l'invention.

La présente invention concerne également l'utilisation de ces composés dans le domaine de la cosmétique.

La présente invention concerne également des compositions galéniques, pharmaceutiques, dermo-cosmétiques ou cosmétiques comprenant un composé tel que défini précédemment.

Ces compositions peuvent se présenter sous l'une des formes connues dans les domaines cosmétiques bien que les formes topique et parentérale soient préférées.

Ces compositions sont utilisables ausi bien dans le domaine humain que vétérinaire.

Un des objets de la présente invention est l'application par voie topique et parentérale des conjugués peptidiques précédents. Les compositions pharmaceutiques peuvent être sous forme administrable par voie orale ou parentérale, injectable notamment, mais de préférence sous forme administrable par voie topique externe.

Ces compositions peuvent être notamment, sous forme de crème, spray, ou lotion par exemple et comporter des excipients connus et éventuellement d'autres principes actifs. Avantageusement, il s'agit de préparations dermo-cosmétologiques sous forme de solutions, de lotions d'émulsions, ou de crèmes utilisées comme accélérateur de bronzage de la peau, sans exposition aux U.V.

Les composés selon la présente invention sont utiles notamment dans la prévention et le traitement des allergies notamment cutanées, des réactions inflammatoires, et des troubles de mélanogénèse.

Les conjugués dont la séquence peptidique possède en position 4 l'amino-acide para-fluoro-Phé (dérivés peptidiques 5 à 8 et 18 à 22), sont particulièrement orientés vers une activité biologique anti-allergique et anti-inflammatoire, par effet suppresseur sur les médiateurs de l'inflammation et de l'allergie (IL1, IL6, $\alpha$TNF, PGE$_2$), à l'exclusion d'une action sur la mélanogénèse.

Les conjugués dont les séquences en position 4 possèdent l'amino-acide D-homo-Phé (dérivés peptidiques 1 à 4 et 9 à 17), sont particulièrement orientés vers des activités de stimulation des processus de mélanogénèse, et possèdent également une activité anti-allergique et anti-inflammatoire.

Les séquences peptidiques selon la présente invention peuvent être obtenues par l'un des procédés quelconque connus de l'homme de métier, notamment par des procédés de synthèse chimique dans laquelle on peut intégrer les différents acides. En tout état de cause compte tenu de la faible dimension des peptides, la synthèse chimique est tout à fait possible et permet d'obtenir des produits très purs.

D'autres caractéristiques des conjugués selon l'invention apparaîtront à la lumière des exemples ci-après, qui illustrent également leur procédé de préparation par synthèse chimique.

**Exemple 1 : Synthèse du conjugué 1 - A = Acide Adipique** Adipoyl - Me.Nle - Glu - His - D-homo-Phé - Arg - Trp - Gly - NH$_2$.

La synthèse a été réalisée par la méthode de Merrifield en phase solide en utilisant une résine MBHA, et comme groupement protecteur FMOC (fluoroényl méthoxy carbonyl).

Les dérivés d'acides aminés utilisés sont les suivants :

FMOC - Gly - OH,

FMOC - Trp - OH,

FMOC - Arg (Tos) - OH,

FMOC - D-homo-Phé - OH,

FMOC - His (Trt) - OH,

FMOC - Glu (chex) - OH,

et

FMOC - Me.Nle - OH,

que l'on couple en utilisant le BOP comme agent de couplage.

Chaque acide aminé est utilisé en excès (x2) ainsi que le BOP (x2) et chaque couplage est répété 2 fois.

L'acide adipique est couplé de façon similaire, le groupement FMOC étant éliminé à chaque étape par la pipéridine (20 % dans le diméthylformamide). La déprotection finale est effectuée en deux étapes :

a) acide trifluoroacétique (2 fois x 5 minutes)
b) acide fluorhydrique anhydre / p-crésol 95,5 (45 minutes).

Le composé brut obtenu avec un rendement de 78 % est repris dans un mélange eau/acide acétique 95,5 % et est ensuite lyophylisé.

Le composé P1 obtenu a une pureté de 82 % (HPLC). 100 mg de ce composé sont ensuite purifiés par HPLC en utilisant une colonne C 18 et on obtient 65 mg de produit pur.

Temps de rétention = 17,04 minutes dans les conditions suivantes :

- colonne C8 (250 mm x 5 mm), détection UV 210 nm
- solvant tampon phosphate Triéthylamine - acétonitrile 10 - 60 % en 15 minutes; débit 1,4 ml /minute.

Analyse des acides aminés
Glu 1,02 - Gly 1,01 - His 1,00 - Arg 0,94 - D-homo-Phé 1,03 - (le Tryptophane n'a pas été déterminé car il se dégrade lors de l'hydrolyse acide).

Spectre de masse (FAB +) 1018,4 / 1126,5

## Exemple 2 : Synthèse du conjugué 5 - A = Acide Adipique

En reprenant le mode opératoire de l'exemple 1 et en remplaçant FMOC-D-homo-Ph-OH par FNIOC-para-fluoro-Phé-OH, on obtient le composé P2 :

Adipoyl - Me.Nle - Glu - His - para-fluoro-Phé - Arg - Trp - Gly - NH$_2$.

F = 120°C, décomposition. $(\alpha)_D$ - 7 (c = 0,4 DMF). HPLC : colonne C$_{18}$, 5 µm, débit 7 mL/min, détection 279 nm, solvant d'élution TFA 0,1 % / acétonitrile 77/23 v/v, temps de rétention 30,45 min. $^1$H RMN (DMSO-d$_6$) ppm : Me. Nle (4,76, 1,62, 1,24, 0,82) ; Glu (8,54, 4,32, 1,86-1,72, 2,24) ; His (8,09, 4,53, 2,95-2,82) ; Para-Fluoro-Phé (8,09, 4,53, 3,00-2,78, 7,3-7,0) ; Arg (8,36, 4,29, 1,54-1,68,1,45, 3,07) ; Trp (8,07, 4,53, 3,16-3,01, 10,80, 7,19, 7,56, 7,30, 6,96) : Gly (8,18, 3-67-3,54).

## Exemple 3 : Synthèse du conjugué 1 - A - Acide trans-10-hydroxy-2-décénoïque

En opérant comme dans l'exemple 1, en remplaçant l'acide adipique par l'acide trans-10-hydroxy-2-décénoïque, on obtient le composé P3:

**trans-10-hydroxy-2-décénoyle - Me.Nle - Glu - His - D-Homo-Phé - Arg - Trp - Gly - NH₂.**

F = 115°C, décomposition. $(\alpha)_D$ - 16 (c = 0,3 DMF). HPLC : colonne $C_{18}$, 5 µm, débit 7 mL/min, détection 279 nm, solvant d'élution TFA 0,1 % / acétonitrile 71/29 v/v, temps de rétention 24,20 min. $^1$H RMN (DMSO-$d_6$) ppm : Ac (1.84) ; Me.Nle (8,00, 4,15, 1,59-1,48, 1,23, 0,83); Glu (8,00, 4,15, 1,86-1,72, 2,19) ; His (8,00, 4,54, 3,00-2,88, 8,89, 7,26); D-Homo-Phé (7,98, 4,53, 2,99-2,78, 7,3-7,0) ; Arg (8,27, 4,29, 1,67-1,53, 1,46, 3,08) ; Trp (8,07, 4,54, 3,16-3,01, 10,80, 7,16, 7,56, 7,30, 7,04, 6,96) ; Gly (8,19, 3,68-3,54).

## Exemple 4 : Synthèse du conjugué 2 - A = Acide α-Amino-Adipique

En opérant comme dans l'exemple 1, en remplaçant l'acide adipique par l'acide α-amino-adipique, on obtient le composé P4 :

**α-amino-adipoyle - Me.Nle - Glu - His - D-Homo-Phé - Arg - Trp - NH₂.**

F = 130°C, décomposition. $(\alpha)_D$ - 5 (c = 0,5 DMF). HPLC : colonne C $_{18}$, 5 µm, débit 7 mL/min, détection 279 nm, solvant d'élution TFA 0,1 % / acétonitrile 77/23 v/v, temps de rétention 28,80 min. $^1$H RMN (DMSO-$d_6$) ppm : Me. Nle (3,77, 1,G2, 1,24. 0.82) : Glu (8,53, 4,32, 1,82-1,71, 2,25); His (8.11, 4,53, 2,98-2,83) ; D-Homo-Phé (8,06, 4,47, 2,99-2,77. 7,25-7, 1) ; Arg (8,32, 4,30, 1,69-1,55, 1,45, 3,07); Trp (8,02, 4,59, 3,16-3,00, 10,78, 7,15, 7,58, 7,30, 7,04, 6,96).

## Exemple 5 : Synthèse du conjugé 3 - A = Acide Sébacique

En reprenant le mode opératoire de l'exemple 1, sans les réactifs FMOC - Gly - OH et FMOC - Trp - OH, en remplaçant l'acide adipique par l'acide sébacique, on obtient le composé P5:

Sébacoyle - Me.Nle - Glu - His - D-Homo-Phé - Arg - NH₂.

F = 130°C, décomposition. $(\alpha)_D$ - 3,2 (c = 0,5 DMF). HPLC : colonne $C_{18}$, 5 µm, débit 7 mL/min, détection 279 nm, solvant d'élution TFA 0,1 % / acétonitrile 77/23 v/v, temps de rétention 21,86 min. $^1$H RMN (DMSO-$d_6$) ppm : Me.Nle (4,70, 1,80, 2,30, 1,95) ; Glu (8,57,4,30, 1,82-1,71. 2,25) ; His (8,21, 4,55, 2,98-2,83) ; Phé (8,09, 4, 55,2,99-2,77, 7,25-7,1) ; Arg (8,31, 4,30, 1,69-1,55, 1,45, 3,07) ; Trp (7,95,4,61, 3,16-3,00, 10,78, 7,15, 7,58, 7,30, 7,04, 6,96) ; Gly (8,32, 3,75).

## Exemple 6 : Etude de l'activité Anti-allergique des conjugués peptidiques, par le test d'Hypersensibilité de contact au Dinitro-fluorobenzène (DNFB).

### Matériel et méthode

Des souris femelles C57 BL/6JICO, âgées de 5 semaines sont réparties en dix lots de dix animaux (cinq par cage), avec libre accès à l'eau et à la nourriture, soumises à une photopériode de douze heures de lumière par vingt-quatre heures.

Les animaux des lots 1 à 6 reçoivent quotidiennement et par application topique sur la peau rasée du dos, les produits à étudier (conjugués 1 et 5 représentés par les composés P1 et P2 préparés selon les exemples 1 et 2), mis en solution dans du propylène glycol, sous un volume constant de 50 µl et cela pendant cinq jours consécutifs.

Posologie par souris et par iour

Composé P1 - Lots 1, 2 et 3 - 2,5 µg, 0,5 µg et 0,1 µg /souris/jour respectivement.
Composé P2 - Lots 4, 5 et 6 - 2,5 µg, 0,5 µg et 0.1 µg /souris/jour respectivement.

Les animaux du lot 10 servent de témoins (ils reçoivent seulement, 50 µl de propylène glycol par application topique pendant cinq jours).

Au cinquième jour, trente minutes après la dernière application toutes les souris sont sensibilisées avec 25 µl de D N F B (2,4-dinitro-1-fluorobenzène (FLUKA CHEMIKA PURUM à 97 % lot n° 33820890)) à 2 % dans un mélange 4 pour 1 d'acétone (SDS réf 0510) et de trioléine (FLUKA) appliqué par voie topique sur la région dorsale de peau rasée.

Au sixième jour, on procède à une nouvelle sensibilisation par application de D N F B.

Au onzième jour, l'épaisseur des oreilles des souris, est mesurée à l'aide d'un micromètre (ROCH 0 à 25 mm au 1/100 mm) afin d'obtenir des valeurs de base, puis les oreilles sont stimulées par application topique de 20 µl de DNFB à 0,8 %.

Au douzième jour, l'épaisseur des oreilles est à nouveau mesurée. On établit la valeur moyenne de l'épaississement des oreilles pour les souris des lots qui ont reçu les produits à étudier, ainsi que pour les souris du lot témoin.

Le pourcentage de suppression éventuelle sera exprimé par le calcul suivant :

$$\frac{T\text{-}E}{T} \; x \; 100$$

avec T pour le pourcentage d'augmentation de l'épaisseur moyenne des oreilles du lot témoin et E pour celui des lots qui ont reçu les produits à étudier.

Les résultats sont rassemblés dans le tableau 1 ci-après.

TABLEAU 1

| Conj. | Lot | % Augment. O. Gauche | % Augment. O. Droite | % Suppress. O. Gauche | % Suppress. O. Droite |
|---|---|---|---|---|---|
| 1 | 1 | 17,50 | 17,80 | 69,80 | 69,57 |
| 1 | 2 | 19,00 | 19,50 | 67,24 | 66,66 |
| 1 | 3 | 21,50 | 22,00 | 62,93 | 62,39 |
| - | T | 58 | 58,50 | - | - |
| 5 | 4 | 17 | 16,65 | 70,48 | 71,39 |
| 5 | 5 | 18,60 | 19,00 | 67,70 | 67,35 |
| 5 | 6 | 20,80 | 21,50 | 63,88 | 63,05 |
| - | T | 57,60 | 58,20 | - | - |

T représente les Lots témoins pour chaque conjugué étudié.

Les conjugués 1 et 5 suppriment de façon hautement significative, de l'ordre de 69 % et 71 %, la réaction d'hypersensibilité cutanée induite par administration de D N F. B dans les conditions expérimentales décrites précédemment.

**Exemple 7 : Etude de l'activité anti-inflammatoire des conjugués 1 et 5 (composés P1 et P2)**

But de l'étude :

Mise en évidence de l'effet inhibiteur des conjugués 1 et 5 (composés P1 et P2) sur la production par des fibroblastes stimulés par l'IL 1, d'un métabolite de l'acide arachidonique, la PGE2.

Méthodes :

**1 -** Culture de fibroblastes pulmonaires embryonnaires d'origine humaine :

Nous avons choisi d'utiliser la souche ATCC MRC5 entretenue au laboratoire de façon continue et déjà testée par Cannon et al. (J. Immunol., 1986). Après un repiquage, les fibroblastes sont cultivés dans des micropuits (plaque de 24 puits de 2 cm$^2$, Falcon). L'innoculum est de 30 000 cellules par puits ; le milieu nutritif utilisé est composé de RPMI 1640 (90 %) et de sérum de veau foetal décomplémenté (SVF, 10 %). Le milieu est changé tous les deux jours jusqu'à confluence des cellules.

2 - Mise en expérience :

Les couches monocellulaires ainsi obtenues sont lavées puis préincubées pendant 24 heures dans du milieu frais ne contenant que 1 % de SVF. Les substances à tester sont ajoutées au milieu à différentes concentrations comprises entre $10^{-6}$ et $10^{-4}$ M. Après 20 minutes d'incubation en présence de ces composés, l'ILI recombinante humaine (Tebu, France) à la concentration de 5, 2,5 ou 0,5 ng/ml de milieu est mise en contact avec des cellules pendant 18 heures. A la fin de cette incubation les surnageants sont prélevés et congelés à - 80 ° C jusqu'à l'analyse. Les couches monocellulaires sont fixées au méthanol.

3 - Dosage de PGE2 :

Le dosage RIA est effectué selon la méthode décrite par Dray et al. (Europ. J. Invest. 1975). 3 séries d'expériences en duplicate ont été réalisées pour chaque concentration de produit étudié et pour les contrôles. Les résultats sont exprimés en pg/µg d'ADN. L'effet inhibiteur est exprimé en pourcentage par rapport aux contrôles.

Afin d'éviter toute erreur possible due au comptage en microscopie optique, l'ADN a été dosé par méthode fluorimétrique selon le protocole décrit par Brunk et al. (Analytical Biochem., 1979).

Les tableaux 2 et 3 ci-après rassemblent une première série d'essais sur la souche MRC5.

TABLEAU 2

| Action du Conjugué 1 | | | |
|---|---|---|---|
| **Concentration IL1 (ng)** | **Concentration Conjugué 1 (M)** | **PGE2 pg/mg d'ADN** | **% inhib.** |
| 2,5 | 0 | $389 \pm 60$ | - |
| | $10^{-4}$ | $179 \pm 50$ | 53 |
| | $10^{-5}$ | $232 \pm 45$ | 40 |
| | $10^{-6}$ | $292 \pm 68$ | 24 |
| | $10^{-7}$ | $364 \pm 68$ | 6 |
| | $10^{-8}$ | $335 \pm 50$ | 13 |
| | $10^{-9}$ | $320 \pm 43$ | 17 |
| 0,5 | 0 | $288 \pm 25$ | - |
| | $10^{-4}$ | $113 \pm 25$ | 60 |
| | $10^{-5}$ | $176 \pm 37$ | 38 |
| | $10^{-6}$ | $231 \pm 55$ | 19 |
| | $10^{-7}$ | $249 \pm 60$ | 13 |
| | $10^{-8}$ | $258 \pm 35$ | 10 |
| | $10^{-9}$ | $364 \pm 56$ | - |

Les résultats montrent une bonne action inhibitrice du conjugué 1 avec un rapport effet dose. Cette action ne dépend pas de la concentration en IL1. On n'observe-pas d'action à partir de $10^{-7}$ M dans les deux cas.

TABLEAU 3

| Action du Conjugué 5 | | | |
|---|---|---|---|
| **Concentration IL1 (ng)** | **Concentration Conjugué 5 (M)** | **PGE2 µg/mg d'ADN** | **% inhib.** |
| 5 | 0 | $7576 \pm 310$ | - |
| | $10^{-4}$ | $2964 \pm 184$ | 60 |
| | $10^{-5}$ | $4580 \pm 230$ | 38 |
| | $10^{-6}$ | $4900 \pm 340$ | 34 |
| | $10^{-7}$ | $6150 \pm 365$ | 17 |
| | $10^{-8}$ | $6142 \pm 255$ | 17 |
| | $10^{-9}$ | $5047 \pm 279$ | 32 |

Les conjugués 1 et 5 possèdent un pouvoir inhibiteur de l'action de l'IL1 et de la PGE2. Très significative cette action pourrait être liée à un blocage des récepteurs.

**Exemple 8 : Etude de l'action des conjugués 1 et 5 sur la mélanogénèse de la souris après l'application topique sous forme de crème dermique (Technique de Warren*)**

Matériel et méthode

- 1) Matériel

- **Excipient dermique N° 66**
    Lano - vaseline - Ac,
    Huile de vaseline Pharmacopée,
    Cire d'abeille Ethoxyle,
    Lanoline purifiée,
    PEG - 200.
- **Formules dermiques**
    Conjugués 1 et 5 (représentés par les composés P1 et P2) incorporés à raison de 10 x $10^{-5}$ M dans l'excipient dermique N° 66.
- **Animaux**
    Souris DBA/2 IFFA - CREDO âgées de 5 semaines.

2) Méthodes (*Raphael WARREN PhD & coll. 0022-202, 1987, Society for Investigate Dermatology*).

- On délimite sur chaque animal une tonsure dorsale après épilation.
- Les produits à essayer sont appliqués par massage à raison de 2 applications par jour, de 50 µl de chaque préparation pendant 5 jours.
- Deux jours après la dernière application, on sacrifie les animaux et on prélève des fractions de 50 mg environ de peau traitée.
- Chaque prélèvement est desséché par lyophilisation et pesé.
- Les fragments de peau sont ensuite soumis à une hydrolyse enzymatique par la protéase K pendant 72 heures à 45°C (Protéase K Merck - 24.568).
- On ajoute aux hydrolysats obtenus 500 µl de $Na_2CO_3$ et 20 µl de $H_2O_2$ à 35 %.
- On met à incuber 30 minutes à 80°C.
- Après refroidissement on ajoute à chaque échantillon 200 µl de chloroforme / méthanol (2 vol/1 vol).
- On centrifuge à 10 000 g.
- On répartit la phase acqueuse (200 µl) dans les puits d'une microplaque "NUNC".

- **Dosage de la Mélanine**

    - Le dosage de la mélanine s'effectue à 405 nm à l'aide d'un Multiskan - Titertek - MCC - 340, comparativement à une gamme étalon de mélanine Sigma M. 8631.

- **Résultats**

Ils sont exprimés en pourcentage de mélanine comparativement aux excipients considérés comme témoins.

**I - Crème 1**-5 $10^{-5}$ M de conjugué 1 pour 50 µl de crème.
**II - Crème 5** - 5 $10^{-5}$ M de conjugué 5 pour 50 µl de crème.
**III - Excipient 66** - comme témoin T.

Les moyennes des résultats sur 15 dosages sont reportés sur le Tableau 4 ci-dessous.

TABLEAU 4

| Echantillon | % Mélanine / témoin |
|---|---|
| I | 80% |
| II | 12 % |
| III | 5% |

Le pourcentage de stimulation est calculé en fonction des valeurs QE et QT, avec QE pour la quantité moyenne de mélanine par mg de peau de l'échantillon testé et QT pour la quantité moyenne de mélanine par mg de peau du témoin, selon la formule

$$\% \text{ de stimulation} = \frac{QE - QT}{QT} \times 100$$

La crème dermique 1 administrée par voie topique, induit de façon hautement significative la synthèse de la Mélanine au niveau de l'épiderme (+ 75 %). Cette activité conforme à l'objet de la présente invention est liée à la structure chimique du conjugué 1 qui contient le groupement D-homo-Phé.

Par contre, la crème dermique 5 administrée par voie topique, n'a pas d'activité sur la stimulation de la mélanogénèse épidermique, cette absence d'activité est liée à la présence dans le conjugué 5 du groupement para-fluoro-Phé.

Ces résultats ont été confirmés par une étude sur la mélanogénèse selon la technique dite de "CLOUDMAN" par culture de mélanocytes in vitro, en utilisant les cellules du mélanome de CLOUDMAN chez le rat.

L'ensemble des résultats obtenus sont conformes à l'objet de la présente invention dont l'objectif est la réalisation de conjugués actifs par voie topique et dont les activités anti allergiques et anti inflammatoires peuvent être séparées de l'activité sur la mélanogénèse selon les applications envisagées.

**Exemple 9 : Etude comparative de l'activité des conjugués et de leurs structures peptidiques, sur l'hypersensibilité de contact au D N F B.**

Matériel et méthode

La méthode utilisée est celle décrite précédemment dans l'exemple N° 4, hypersensibilité de contact induite chez la souris par la D N F B.

- **Produits étudiés**

Lot I : **trans-10-hydroxy-2-décénoyl - Me-Nle - Glu - His - D-homo-Phé - Arg - Trp - Gly - NH$_2$**

- Conjugué 1 représenté par le composé P3.
Lot II : Me.Nle - Glu - His - D-homo-Phé - Arg - Trp - Gly - NH$_2$
Lot T : Témoin = Acide trans-10-hydroxy-2-décénoïque.

Solvant : le propylène glycol précédemment employé comme témoin dans l'exemple 6.

Une mesure de l'activité du témoin sera effectuée pour chaque expérimentation.

- **Expérimentation et posologie**

| Lot I : Doses utilisées | Lot II : Doses utilisées |
|---|---|
| 1 = 0,1 µg/souris/jour | 4 = 0,1 µg/souris/jour |
| 2 = 0,5 µg/souris/jour | 5 = 0,5 µg/souris/jour |
| 3 = 2,5 µg/souris/jour | 6 = 2,5 µg/souris/jour |

Témoin: 10 ng d' acide trans-10-hydroxy-2-décénoïque par souris et par jour.

Au cinquième jour, trente minutes après la dernière application toutes les souris sont sensibilisées avec 25 μl de D N F B (2.4-dinitro-1-fluorobenzène (FLUKA CHEMIKA PURUM à 97 % lot N° 33820890)) à 2 % dans un mélange 4 pour 1 d'acétone (SDS réf. 05510) et de trioléine (FLUKA) appliqué par voie topique sur la région dorsale de peau rasée. Au sixième jour, on procède à une nouvelle sensibilisation par application de D N F B. Au onzième jour, l'épaisseur des oreilles des souris, est mesurée à l'aide d'un micromètre (ROCH 0 à 25 mm au 1/100 mm) afin d'obtenir des valeurs de base, puis les oreilles sont stimulées par application topique de 20 μl de D N F B à 0,8 %. Au douzième jour, l'épaisseur des oreilles est à nouveau mesurée. On établit la valeur moyenne de l'épaississement des oreilles pour les souris des lots qui ont reçu les produits à étudier, ainsi que pour les souris du lot témoin.Le pourcentage de suppression éventuelle sera exprimé par la même équation que dans l'exemple 6.

Les résultats sont reportés dans le Tableau 5 ci-après.

TABLEAU 5

| Lot | Dose* | % Augment. O. Gauche | % Augment. O. Droite | % Suppress. O. Gauche | % Suppress. O. Droite |
|---|---|---|---|---|---|
| I | 2,5 | 11,00 | 11,40 | 80,28 | 79,64 |
| I | 0.5 | 13,00 | 12,90 | 76,70 | 76,96 |
| I | 0,1 | 15,60 | 14,60 | 72,04 | 73,92 |
| T | 0,01 | 55,80 | 56,00 | - | - |
| II | 2,5 | 17,60 | 18,50 | 69,17 | 67,82 |
| II | 0,5 | 24,00 | 22,80 | 57,96 | 60,34 |
| II | 0,1 | 32,00 | 30,20 | 43,95 | 47,47 |
| T | 0,01 | 57,10 | 57,50 | - | - |

* μg/souris/jour.

On observe 80 % de suppression de l'hypersensibilité cutanée à la dose de 2,5 μg/souris/jour pour le conjugué 1 (composé P3) selon l'invention, et une nette relation doses/effets entre les doses de 0,1, 0,5 et 2,5 μg/souris/jour. Par contre, pour le peptide seul, on observe 47 % de suppression de l'hypersensibilité cutanée à la dose de 0,1 μg/souris/jour et, pour les doses de 0,5 μg et de 2,5 μg, la réponse suppressive est de l'ordre de 60 et 69 %. Enfin, on n'observe pas d'effet suppressif de l'hypersensibilité cutanée par administration de l'acide trans-10-hydroxy-2-décénoïque, en solution dans le propylène - glycol.

Administrés par voie topique et dans les conditions expérimentales décrites, le conjugué 1 selon l'invention induit de façon hautement significative la suppression de l'hypersensibilité cutanée chez la souris.

LISTE DE SEQUENCES

(1) INFORMATIONS GENERALES:

(i) DEPOSANT:

(A) NOM: INSTITUT EUROPEEN DE BIOLOGIE CELLULAIRE
(B) RUE: 18 AVENUE DE L'EUROPE
(C) VILLE: RAMONVILLE-ST-AGNE
(E) PAYS: FRANCE
(F) CODE POSTAL: 31520

(ii) TITRE DE L'INVENTION: CONJUGUES D'ALPHA-MSH AVEC UN ACIDE GRAS, LEUR PROCEDE DE PREPARATION ET LEUR UTILISATION A TITRE DE MEDICAMENT

(iii) NOMBRE DE SEQUENCES: 23

(iv) FORME DECHIFFRABLE PAR ORDINATEUR:

    (A) TYPE DE SUPPORT: Floppy disk
    (B) ORDINATEUR: IBM PC compatible
    (C) SYSTEME D' EXPLOITATION: PC-DOS/MS-DOS
    (D) LOGICIEL: PatentIn Release #1.0, Version #1.30 (OEB)

(v) DONNEES DE LA DEMANDE ACTUELLE: NUMERO DE LA DEMANDE: FR 9600890

(2) INFORMATIONS POUR LA SEQ ID NO: 1:

    (i) CARACTERISTIQUES DE LA SEQUENCE:

        (A) LONGUEUR: 4 acides aminés
        (B) TYPE: acide aminé
        (C) NOMBRE DE BRINS:
        (D) CONFIGURATION: linéaire

    (ii) TYPE DE MOLECULE: peptide

    (xi) DESCRIPTION DE LA SEQUENCE: SEQ ID NO: 1:

<div align="center">

**Glu His Phe Arg**
1

</div>

(2) INFORMATIONS POUR LA SEQ ID NO: 2:

    (i) CARACTERISTIQUES DE LA SEQUENCE:

        (A) LONGUEUR: 7 acides aminés
        (B) TYPE: acide aminé
        (C) NOMBRE DE BRINS:
        (D) CONFIGURATION: linéaire

    (ii) TYPE DE MOLECULE: peptide

    (ix) CARACTERISTIQUE:

        (A) NOM/CLE: Modified-site
        (B) EMPLACEMENT: 1
        (D) AUTRES INFORMATIONS: "Xaa signifie 5-Me-Norleucine"

    (ix) CARACTERISTIQUE:

        (A) NOM/CLE: Modified-site
        (B) EMPLACEMENT:4
        (D) AUTRES INFORMATIONS: "Xaa signifie D-homo-Phe"

    (ix) CARACTERISTIQUE:

        (A) NOM/CLE: Modified-site
        (B) EMPLACEMENT:7
        (D) AUTRES INFORMATIONS: "Xaa signifie Gly-NH2"

    (xi) DESCRIPTION DE LA SEQUENCE: SEQ ID NO: 2:

Xaa Glu His Xaa Arg Trp Xaa
1                              5

(2) INFORMATIONS POUR LA SEQ ID NO: 3:

(i) CARACTERISTIQUES DE LA SEQUENCE:

(A) LONGUEUR: 6 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT:1
(D) AUTRES INFORMATIONS: "Xaa signifie 5-Me-norleucine"

(ix) CARACTERISTIQUE:

(A) NOM/CLE: Modified-site
(B) EMPLACEMENT:4
(D) AUTRES INFORMATIONS: "Xaa signifie D-homo-Phe"

(ix) CARACTERISTIQUE:

(A) NOM/CLE: Modified-site
(B) EMPLACEMENT:6
(D) AUTRES INFORMATIONS: "Xaa signifie Trp-NH2"

(xi) DESCRIPTION DE LA SEQUENCE: SEQ ID NO: 3: :

Xaa Glu His Xaa Arg Xaa
1                          5

(2) INFORMATIONS POUR LA SEQ ID NO: 4:

(i) CARACTERISTIQUES DE LA SEQUENCE:

(A) LONGUEUR: 5 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT:1
(D) AUTRES INFORMATIONS: "Xaa signifie 5-Me-Norleucine"

(ix) CARACTERISTIQUE:

       (A) NOM/CLE: Modified-site
       (B) EMPLACEMENT:4
       (D) AUTRES INFORMATIONS: "Xaa signifie D-homo-Phe"

    (ix) CARACTERISTIQUE:

       (A) NOM/CLE: Modified-site
       (B) EMPLACEMENT:5
       (D) AUTRES INFORMATIONS: "Xaa signifié Arg-NH2"

    (xi) DESCRIPTION DE LA SEQUENCE: SEQ ID NO: 4:

$$\text{Xaa Glu His Xaa Xaa}$$
$$1 \qquad\qquad\qquad 5$$

(2) INFORMATIONS POUR LA SEQ ID NO: 5:

    (i) CARACTERISTIQUES DE LA SEQUENCE:

       (A) LONGUEUR: 4 acides aminés
       (B) TYPE: acide aminé
       (C) NOMBRE DE BRINS:
       (D) CONFIGURATION: linéaire

    (ii) TYPE DE MOLECULE: peptide

    (ix) CARACTERISTIQUE:

       (A) NOM/CLE: Modified-site
       (B) EMPLACEMENT: 1
       (D) AUTRES INFORMATIONS: "Xaa signifie 5-Me-Norleucine"

    (ix) CARACTERISTIQUE:

       (A) NOM/CLE: Modified-site
       (B) EMPLACEMENT:4
       (D) AUTRES INFORMATIONS: "Xaa signifie D-homo-Phe-NH2"

    (xi) DESCRIPTION DE LA SEQUENCE: SEQ ID NO: 5:

$$\text{Xaa Glu His Xaa}$$
$$1$$

(2) INFORMATIONS POUR LA SEQ ID NO: 6:

    (i) CARACTERISTIQUES DE LA SEQUENCE:

       (A) LONGUEUR: 7 acides aminés
       (B) TYPE: acide aminé
       (C) NOMBRE DE BRINS:
       (D) CONFIGURATION: linéaire

    (ii) TYPE DE MOLECULE: peptide

    (ix) CARACTERISTIQUE:

(A) NOM/CLE: Modified-site
(B) EMPLACEMENT:1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(ix) CARACTERISTIQUE:

(A) NOM/CLE: Modified-site
(B) EMPLACEMENT:7
(D) AUTRES INFORMATIONS: "Xaa signifie Gly-NH2"

(xi) DESCRIPTION DE LA SEQUENCE: SEQ ID NO: 6:

$$\text{Xaa Glu His Xaa Arg Trp Xaa}$$
$$1 \qquad\qquad 5$$

(2) INFORMATIONS POUR LA SEQ ID NO: 7:

(i) CARACTERISTIQUES DE LA SEQUENCE:

(A) LONGUEUR: 6 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(ix) CARACTERISTIQUE:

(A) NOM/CLE: Modified-site
(B) EMPLACEMENT:6
(D) AUTRES INFORMATIONS: "Xaa signifie Trp-NH2"

(xi) DESCRIPTION DE LA SEQUENCE: SEQ ID NO: 7:

$$\text{Xaa Glu His Xaa Arg Xaa}$$
$$1 \qquad\qquad 5$$

(2) INFORMATIONS POUR LA SEQ ID NO: 8:

(i) CARACTERISTIQUES DE LA SEQUENCE:

(A) LONGUEUR: 5 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(ix) CARACTERISTIQUE:

(A) NOM/CLE: Modified-site
(B) EMPLACEMENT:5
(D) AUTRES INFORMATIONS: "Xaa signifie Arg-NH2"

(xi) DESCRIPTION DE LA SEQUENCE: SEQ ID NO: 8:

Xaa Glu His Xaa Xaa
1                    5

(2) INFORMATIONS POUR LA SEQ ID NO: 9:

(i) CARACTERISTIQUES DE LA SEQUENCE:

(A) LONGUEUR: 4 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT:1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe-NH2"

(xi) DESCRIPTION DE LA SEQUENCE: SEQ ID NO: 9:

<div align="center">

Xaa Glu His Xaa

1

</div>

(2) INFORMATIONS POUR LA SEQ ID NO: 10:

    (i) CARACTERISTIQUES DE LA SEQUENCE:

        (A) LONGUEUR: 7 acides aminés
        (B) TYPE: acide aminé
        (C) NOMBRE DE BRINS:
        (D) CONFIGURATION: linéaire

    (ii) TYPE DE MOLECULE: peptide

    (ix) CARACTERISTIQUE:

        (A) NOM/CLE: Modified-site
        (B) EMPLACEMENT: 1
        (D) AUTRES INFORMATIONS: "Xaa signifie 2-N-Me-Norleucine"

    (ix) CARACTERISTIQUE:

        (A) NOM/CLE: Modified-site
        (B) EMPLACEMENT:4
        (D) AUTRES INFORMATIONS: "Xaa signifie D-homo-Phe"

    (ix) CARACTERISTIQUE:

        (A) NOM/CLE: Modified-site
        (B) EMPLACEMENT:7
        (D) AUTRES INFORMATIONS: "Xaa signifie Gly-NH2"

    (xi) DESCRIPTION DE LA SEQUENCE: SEQ ID NO: 10:

<div align="center">

Xaa Glu His Xaa Arg Trp Xaa

1          5

</div>

(2) INFORMATIONS POUR LA SEQ ID NO: 11:

    (i) CARACTERISTIQUES DE LA SEQUENCE:

        (A) LONGUEUR: 6 acides aminés
        (B) TYPE: acide aminé
        (C) NOMBRE DE BRINS:
        (D) CONFIGURATION: linéaire

    (ii) TYPE DE MOLECULE: peptide

    (ix) CARACTERISTIQUE:

        (A) NOM/CLE: Modified-site
        (B) EMPLACEMENT: 1
        (D) AUTRES INFORMATIONS: "Xaa signifie 2-N-Me-Norleucine"

    (ix) CARACTERISTIQUE:

(A) NOM/CLE: Modified-site
(B) EMPLACEMENT:4
(D) AUTRES INFORMATIONS: "Xaa signifie D-homo-Phe"

(ix) CARACTERISTIQUE:

(A) NOM/CLE: Modified-site
(B) EMPLACEMENT:6
(D) AUTRES INFORMATIONS: "Xaa signifie Trp-NH2"

(xi) DESCRIPTION DE LA SEQUENCE: SEQ ID NO: 11:

Xaa Glu His Xaa Arg Xaa
1                    5

(2) INFORMATIONS POUR LA SEQ ID NO: 12:

(i) CARACTERISTIQUES DE LA SEQUENCE:

(A) LONGUEUR: 5 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT:1
(D) AUTRES INFORMATIONS: "Xaa signifie 2-N-Me-Norleucine"

(ix) CARACTERISTIQUE:

(A) NOM/CLE: Modified-site
(B) EMPLACEMENT:4
(D) AUTRES INFORMATIONS: "Xaa signifie D-homo-Phe"

(ix) CARACTERISTIQUE:

(A) NOM/CLE: Modified-site
(B) EMPLACEMENT:5
(D) AUTRES INFORMATIONS: "Xaa signifie Arg-NH2"

(xi) DESCRIPTION DE LA SEQUENCE: SEQ ID NO: 12:

Xaa Glu His Xaa Xaa
1                 5

(2) INFORMATIONS POUR LA SEQ ID NO: 13:

(i) CARACTERISTIQUES DE LA SEQUENCE:

(A) LONGUEUR: 4 acides aminés
(B) TYPE: acide aminé
(C) NOMBRE DE BRINS:

(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 2-N-Me-Norleucine"

(ix) CARACTERISTIQUE:

(A) NOM/CLE: Modified-site
(B) EMPLACEMENT:4
(D) AUTRES INFORMATIONS: "Xaa signifie D-homo-Phe-NH2"

(xi) DESCRIPTION DE LA SEQUENCE: SEQ ID NO: 13: :

Xaa Glu His Xaa
1

(2) INFORMATIONS POUR LA SEQ ID NO: 14:

(i) CARACTERISTIQUES DE LA SEQUENCE:

(A) LONGUEUR: 7 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie D-homo-Phe"

(xi) DESCRIPTION DE LA SEQUENCE: SEQ ID NO: 14:

Xaa Glu His Xaa Arg Trp Gly
1                      5

(2) INFORMATIONS POUR LA SEQ ID NO: 15:

(i) CARACTERISTIQUES DE LA SEQUENCE:

(A) LONGUEUR: 6 acides aminés
(B) TYPE: acide aminé
(C) NOMBRE DE BRINS:

(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie D-homo-Phe"

(xi) DESCRIPTION DE LA SEQUENCE: SEQ ID NO: 15:

Xaa Glu His Xaa Arg Trp
1               5

(2) INFORMATIONS POUR LA SEQ ID NO: 16:

(i) CARACTERISTIQUES DE LA SEQUENCE:

(A) LONGUEUR: 5 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT:1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie D-homo-Phe"

(xi) DESCRIPTION DE LA SEQUENCE: SEQ ID NO: 16:

Xaa Glu His Xaa Arg
1               5

(2) INFORMATIONS POUR LA SEQ ID NO: 17:

(i) CARACTERISTIQUES DE LA SEQUENCE:

(A) LONGUEUR: 7 acides aminés
(B) TYPE: acide aminé
(C) NOMBRE DE BRINS:

(D) CONFIGURATION: linéaire

(ii) TYPE DE MOLECULE: peptide

(ix) CARACTERISTIQUE:

    (A) NOM/CLE: Modified-site
    (B) EMPLACEMENT: 1
    (D) AUTRES INFORMATIONS: "Xaa signifie 2-N-Me-Norleucine"

(ix) CARACTERISTIQUE:

    (A) NOM/CLE: Modified-site
    (B) EMPLACEMENT:4
    (D) AUTRES INFORMATIONS: "Xaa signifie D-homo-Phe"

(xi) DESCRIPTION DE LA SEQUENCE: SEQ ID NO: 17:

$$\underset{1}{Xaa}\ Glu\ His\ \underset{5}{Xaa}\ Arg\ Trp\ Gly$$

(2) INFORMATIONS POUR LA SEQ ID NO: 18:

    (i) CARACTERISTIQUES DE LA SEQUENCE:

        (A) LONGUEUR: 6 acides aminés
        (B) TYPE: acide aminé
        (C) NOMBRE DE BRINS:
        (D) CONFIGURATION: linéaire

    (ii) TYPE DE MOLECULE: peptide

    (ix) CARACTERISTIQUE:

        (A) NOM/CLE: Modified-site
        (B) EMPLACEMENT: 1
        (D) AUTRES INFORMATIONS: "Xaa signifie 2-N-Me-Norleucine"

    (ix) CARACTERISTIQUE:

        (A) NOM/CLE: Modified-site
        (B) EMPLACEMENT:4
        (D) AUTRES INFORMATIONS: "Xaa signifie D-homo-Phe"

    (xi) DESCRIPTION DE LA SEQUENCE: SEQ ID NO: 18:

$$\underset{1}{Xaa}\ Glu\ His\ \underset{5}{Xaa}\ Arg\ Trp$$

(2) INFORMATIONS POUR LA SEQ ID NO: 19:

    (i) CARACTERISTIQUES DE LA SEQUENCE:

        (A) LONGUEUR: 7 acides aminés
        (B) TYPE: acide aminé
        (C) NOMBRE DE BRINS:

(D) CONFIGURATION: linéaire

(ii) TYPE DE MOLECULE: peptide

(ix) CARACTERISTIQUE:

(A) NOM/CLE: Modified-site
(B) EMPLACEMENT:1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(xi) DESCRIPTION DE LA SEQUENCE: SEQ ID NO: 19:

Xaa Glu His Xaa Arg Trp Gly
1                 5

(2) INFORMATIONS POUR LA SEQ ID NO: 20:

(i) CARACTERISTIQUES DE LA SEQUENCE:

(A) LONGUEUR: 6 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(xi) DESCRIPTION DE LA SEQUENCE: SEQ ID NO: 20:

Xaa Glu His Xaa Arg Trp
1                 5

(2) INFORMATIONS POUR LA SEQ ID NO: 21:

(i) CARACTERISTIQUES DE LA SEQUENCE:

(A) LONGUEUR: 5 acides aminés
(B) TYPE: acide aminé
(C) NOMBRE DE BRINS:

(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 5-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(xi) DESCRIPTION DE LA SEQUENCE: SEQ ID NO: 21:

Xaa Glu His Xaa Arg
1                         5

(2) INFORMATIONS POUR LA SEQ ID NO: 22:

(i) CARACTERISTIQUES DE LA SEQUENCE:

(A) LONGUEUR: 7 acides aminés
(B) TYPE: acide aminé
(C) NOMBRE DE BRINS:
(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 2-N-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(xi) DESCRIPTION DE LA SEQUENCE: SEQ ID NO: 22: :

Xaa Glu His Xaa Arg Trp Gly
1                         5

(2) INFORMATIONS POUR LA SEQ ID NO: 23:

(i) CARACTERISTIQUES DE LA SEQUENCE:

(A) LONGUEUR: 6 acides aminés
(B) TYPE: acide aminé
(C) NOMBRE DE BRINS:

(D) CONFIGURATION: linéaire

(ii) TYPE DE MOLECULE: peptide

(ix) CARACTERISTIQUE:

(A) NOM/CLE: Modified-site
(B) EMPLACEMENT: 1
(D) AUTRES INFORMATIONS: "Xaa signifie 2-N-Me-Norleucine"

(ix) CARACTERISTIQUE:

(A) NOM/CLE: Modified-site
(B) EMPLACEMENT:4
(D) AUTRES INFORMATIONS: "Xaa signifie para-fluoro-Phe"

(xi) DESCRIPTION DE LA SEQUENCE: SEQ ID NO: 23:

$$\text{Xaa Glu His Xaa Arg Trp}$$
$$1 \qquad\qquad 5$$

## Revendications

1. Conjugué peptidique, **caractérisé en ce qu'**il s'agit de conjugués de formule générale III :

$$A - X - Glu - His - Phé - Y \quad (III)$$

dans laquelle
X représente un groupe 5-Me.Norleucine (Me.Nle), 2-N-Me.Norceuline (N-Me.Nle), OH ou $NH_2$,
Y représente un reste Arg - Z ou Z dans lequel Z est Trp - Gly - OH, Trp - OH, OH, Trp - Gly - $NH_2$, Trp - $NH_2$, ou $NH_2$,
Phé représente un reste D-Homo-Phé ou para-fluoro-Phé,
les acides aminés étant sous forme D, L ou D,L, ladite séquence étant conjuguée, chimiquement ou physiquement avec des acides A sélectionnés parmi :

- les acides di-carboxyliques de formule générale I

$$HOOC - R1 - COOH \qquad\qquad (I)$$

dans laquelle R1 représente un radical alkylène, linéaire ou ramifié, comprenant au moins 3 atomes de carbone, de préférence de 3 à 10 atomes de carbone, éventuellement substitué, notamment par un ou plusieurs groupes amino ou hydroxy, et
- les acides gras $\alpha$-monoinsaturés de configuration cis ou trans, de préférence trans, de formule générale II

$$R2 - CH = CH - COOH \qquad\qquad (II)$$

dans laquelle R2 représente un radical alkyle, linéaire ou ramifié comprenant au moins 6 atomes de carbone, de préférence de 6 à 10 atomes de carbone, substitué par un groupe amino, hydroxy ou oxo.

2. Conjugué selon la revendication 1, **caractérisé en ce que** la séquence d'acides aminés est liée à l'acide sélectionné sous forme de sel, d'ester ou d'amide.

3. Conjugué selon l'une des revendications 1 ou 2, **caractérisé en ce que** les acides de formules générales I ou II sont de préférence choisis parmi les diacides carboxyliques de formule pour lesquels R1 représente un reste alkylène en $C_4$-$C_8$, substitué ou non, en particulier les acides $\alpha$-adipiques, $\alpha$-amino-adipiques et dérivés, l'acide sébacique et dérivés, les acides gras $\alpha$-monoinsaturés de formule II pour lesquels R2 représente un reste alkyle, linéaire ou ramifié en $C_7$, en particulier les acides hydroxydécénoïques, et décénoïliques, sous forme des sels, esters, ou amides correspondants.

4.  Conjugué selon la revendication 3, **caractérisé en ce que** les acides de formules générales I ou II sont de préférence choisis parmi l'acide adipique, l'acide $\alpha$-amino adipique, l'acide sébacique, l'acide trans - 10 - hydroxy - $\Delta^2$ - décénoïque et l'acide trans-oxo - 9 - décéne - 2 - oïque.

5.  Conjugué selon l'une des revendications 1 à 4, **caractérisé en ce qu'**il est sélectionné parmi les dérivés peptidiques suivants :

1   A - Me.Nle - Glu - His - D-homo-Phé - Arg - Trp - Gly - NH$_2$

2   A - Me.Nle - Glu - His - D-homo-Phé - Arg - Trp - NH$_2$

3   A - Me.Nle - Glu - His - D-homo-Phé - Arg - NH$_2$

4   A - Me.Nle - Glu - His - D-homo-Phé - NH$_2$

5   A - Me.Nle - Glu - His - para-fluoro-Phé - Arg - Trp - Gly - NH$_2$

6   A - Me.Nle - Glu - His - para-fluoro-Phé - Arg - Trp - NH$_2$

7   A - Me.Nle - Glu - His - para-fluoro-Phé - Arg - NH$_2$

8   A - Me.Nle - Glu - His - para-fluoro-Phé - NH$_2$

9   A.- N - Me.Nle - Glu - His - D-homo-Phé - Arg - Trp - Gly - NH$_2$

10   A - N - Me.Nle - Glu - His - D-homo-Phé- Arg - Trp - NH$_2$

11   A - N - Me.Nle - Glu - His - D-homo-Phé - Arg - NH$_2$

12   A - N - Me.Nle - Glu - His - D-homo-Phé - NH$_2$

13   A - Me.Nle - Glu - His - D-homo-Phé - Arg - Trp - Gly - OH

14   A - Me.Nle - Glu - His - D-homo-Phé - Arg - Trp - OH

15   A - Me.Nle - Glu - His - D-homo-Phé - Arg - OH

16      A - N - Me.Nle - Glu - His - D-homo-Phé - Arg - Trp - Gly - OH

17      A - N - Me.Nle - Glu - His - D-homo-Phé - Arg - Trp - OH

18      A - Me.Nle - Glu - His - para-fluoro-Phé - Arg - Trp - Gly - OH

19      A - Me.Nle - Glu - His - para-fluoro-Phé - Arg - Trp - OH

20      A - Me.Nle - Glu - His - para-fluoro-Phé - Arg - OH

21      A - N - Me.Nle - Glu - His - para-fluoro-Phé - Arg - Trp - Gly - OH

22      A - N - Me.Nle - Glu - His - para-fluoro-Phé - Arg - Trp - OH

A étant un acide de formules générales I ou II telles que définies dans les revendications 1, 3 et 4, ainsi que les dérivés de ces molécules sous forme de sels d'esters ou d'amides.

**6.** A titre de médicaments, les conjugués selon l'une des revendications 1 à 5.

**7.** Médicament selon la revendication 6, utile pour le traitement des allergies notamment cutanées, des réactions inflammatoires et des troubles de la mélanogénèse, utilisable en médecine humaine et vétérinaire.

**8.** Composition galénique, **caractérisée en ce qu'**elle comprend un conjugué selon l'une des revendications 1 à 5.

**9.** Composition galénique selon la revendications 8, **caractérisée en ce qu'**il s'agit de préparations dermo-cosmétologiques sous forme de solutions, de lotions d'émulsions, ou de crèmes utilisées comme accélérateur de bronzage de la peau, sans exposition aux U.V.

**10.** Composition galénique selon l'une des revendications 8 ou 9, **caractérisée en ce qu'**il s'agit de préparations galéniques, sous forme de solutions, de lotions, de crème, de spray.

**Claims**

**1.** Peptide conjugate, **characterized in that** it includes peptide conjugates of general formula III:

$$\text{A–X–Glu–His–Phe–Y (III)}$$

in which
X represents a 5-Me.Norleucine (Me.Nle), 2-N-Me-Norleucine (N-Me.Nle) , OH or $NH_2$ group,
Y represents a residue Arg-Z or Z in which Z is Trp-Gly-OH, Trp-OH, OH, Trp-Gly-$NH_2$, Trp-$NH_2$, or $NH_2$,
Phe represents a D-Homo-Phe or para-fluoro-Phe residue,
the amino acids being in the D, L or D,L form, the said sequence being chemically or physically conjugated with acids A selected from :

-  dicarboxylic acids of general formula I

HOOC-R1-COOH                                         (I)

in which R1 represents a straight or branched alkylene radical comprising at least 3 carbon atoms, preferably having from 3 to 10 carbon atoms, optionally substituted in particular by one or more amino or hydroxyl groups, and

- $\alpha$-monounsaturated fatty acids of cis or trans, preferably trans, configuration, of general formula II

$$R2\text{-}CH = CH\text{-}COOH \qquad\qquad (II)$$

in which R2 represents a straight or branched alkyl radical comprising at least 6 carbon atoms, preferably from 6 to 10 carbon atoms, substituted by an amino, hydroxyl or oxo group.

2. Conjugate according to Claim 1, **characterized in that** the amino acid sequence is linked to the acid selected in the form of a salt, ester or amide.

3. Conjugate according to either of Claims 1 and 2, **characterized in that** the acids of general formulae I or II are preferably chosen from dicarboxylic acids of formula I for which R1 represents a substituted or unsubstituted $C_4$-$C_8$ alkylene residue, in particular $\alpha$-adipic and $\alpha$-aminoadipic acids and derivatives, sebacic acid and derivatives, $\alpha$-monounsaturated fatty acids of formula II for which R2 represents a straight or branched $C_7$ alkyl residue, in particular hydroxydecenoic and decenoilic acids in the form of the corresponding salts, esters or amides.

4. Conjugate according to Claim 3, **characterized in that** the acids of general formulae I or II are preferably chosen from adipic acid, $\alpha$-aminoadipic acid, sebacic acid, trans-10-hydroxy-$\Delta^2$-decenoic acid and trans-9-oxo-2-decenoic acid.

5. Conjugate according to one of Claims 1 to 4, **characterized in that** it is selected from the following peptide derivatives:

```
1     A-Me.Nle-Glu-His-D-homo-Phe-Arg-Trp-Gly-NH₂

2     A-Me.Nle-Glu-His-D-homo-Phe-Arg-Trp-NH₂

3     A-Me.Nle-Glu-His-D-homo-Phe-Arg-NH₂

4     A-Me.Nle-Glu-His-D-homo-Phe-NH₂

5     A-Me.Nle-Glu-His-para-fluoro-Phe-Arg-Trp-Gly-NH₂

6     A-Me.Nle-Glu-His-para-fluoro-Phe-Arg-Trp-NH₂

7     A-Me.Nle-Glu-His-para-fluoro-Phe-Arg-NH₂

8     A-Me.Nle-Glu-His-para-fluoro-Phe-NH₂

9     A-N-Me.Nle-Glu-His-D-homo-Phe-Arg-Trp-Gly-NH₂

10    A-N-Me.Nle-Glu-His-D-homo-Phe-Arg-Trp-NH₂
```

```
11    A-N-Me.Nle-Glu-His-D-homo-Phe-Arg-NH₂

12    A-N-Me.Nle-Glu-His-D-homo-Phe-NH₂

13    A-Me.Nle-Glu-His-D-homo-Phe-Arg-Trp-Gly-OH

14    A-Me.Nle-Glu-His-D-homo-Phe-Arg-Trp-OH

15    A-Me.Nle-Glu-His-D-homo-Phe-Arg-OH

16    A-N-Me.Nle-Glu-His-D-homo-Phe-Arg-Trp-Gly-OH

17    A-N-Me.Nle-Glu-His-D-homo-Phe-Arg-Trp-OH

18    A-Me.Nle-Glu-His-para-fluoro-Phe-Arg-Trp-Gly-OH

19    A-Me.Nle-Glu-His-para-fluoro-Phe-Arg-Trp-OH

20    A-Me.Nle-Glu-His-para-fluoro-Phe-Arg-OH

21    A-N-Me.Nle-Glu-His-para-fluoro-Phe-Arg-Trp-Gly-OH

22    A-N-Me.Nle-Glu-His-para-fluoro-Phe-Arg-Trp-OH
```

A being an acid of general formulae I or II as defined in Claims 1, 3 and 4, as well as the derivatives of these molecules in the form of ester or amide salts.

6. As drugs, the conjugates according to one of Claims 1 to 5.

7. Drug according to Claim 6, useful for the treatment of allergies, in particular skin allergies, inflammatory reactions and melanogenesis disorders, which can be used in human and veterinary medicine.

8. Galenic composition, **characterized in that** it comprises a conjugate according to one of Claims 1 to 5.

9. Galenic composition according to Claim 8, **characterized in that** they are dermocosmetic preparations in the form of solutions, lotions, emulsions or creams which are used as skin tanning accelerator, without exposure to UV radiation.

10. Galenic composition according to either of Claims 8 and 9, **characterized in that** they are galenic preparations, in the form of solutions, lotions, a cream or a spray.

**Patentansprüche**

1. Peptid-Konjugat, **dadurch gekennzeichnet, dass** es sich um Konjugate der allgemeinen Formel III :

$$A - X - Glu - His - Phe - Y \qquad (III)$$

handelt, worin:

X eine 5-Me.Norleucin- (Me.Nle), 2-N-Me.Norleucin- (N-Me.Nle), OH oder $NH_2$ Gruppe darstellt;

Y einen Arg-Z- oder Z-Rest darstellt, worin Z Trp-Gly-OH, Trp-OH, OH, Trp-Gly-$NH_2$, TrP-$NH_2$ oder $NH_2$ ist,

Phe einen D-Homo-Phe- oder para-Fluor-Phe-Rest darstellt,

worin die Aminosaüren in der D-, L- oder D,L-Form sind,
worin die Sequenz chemisch oder physikalisch mit Säuren A konjugiert ist, ausgewählt unter:

den Dicarbonsäuren der allgemeinen Formel I

$$HOOC-R1-COOH \qquad (I)$$

worin R1 einen linearen oder verzweigten Alkenylrest mit mindestens 3 Kohlenstoffatome, vorzugsweise 3 bis 10 Kohlenstoffatome darstellt, welcher optional substituiert ist, und insbesondere mit einer oder mehreren Amino- oder Hydroxy-Ggruppen substituiert ist, und
den $\alpha$ einfach ungesättigten Fettsäuren in cis- oder trans-, vorzugsweise trans-Konfiguration - der allgemeinen Formel (II)

$$R2 - CH=CH-COOH \qquad (II)$$

worin R2 einen linearen oder verzweigten Alkylrest mit mindestens 6 Kohlenstoffatomen, vorzugsweise 6 bis 10 Kohlenstoffatomen darstellt, welcher mit einer Amino-, Hydroxy- oder Oxo-Gruppe substituiert ist.

**2.** Konjugat nach Anspruch 1 **dadurch gekennzeichnet, dass** die Aminosäuresequenz in Form eines Salzes, Esters oder Amids an der Säure verbunden ist.

**3.** Konjugat nach einem der Ansprüche 1 oder 2, **dadurch gekennzeichnet, dass** die Säuren der allgemeinen Formeln I oder II vorzugsweise ausgewählt sind, aus den Di-carbonsäuren der Formel I, worin R1 einen substituierten oder nicht substituierten $C_4$-$C_8$ Alkylenrest, insbesondere die $\alpha$-Adipinsäuren, die $\alpha$-Amino-adipinsaüren und deren Derivate, die Sebacinsäure und derer Derivate darstellt, und den $\alpha$-einfach ungesättigten Fettsäuren der allgemeinen Formel II, worin R2 einen linearen oder verzweigten $C_7$-Alkylrest, insbesondere die Hydroxydecenoyl- und Decenoyl-Säuren in Form der entsprechenden Salze, Ester oder Amide darstellt.

**4.** Konjugat nach Anspruch 3, **dadurch gekennzeichnet, dass** die Säuren der allgemeinen Formeln I oder II vorzugsweise ausgewählt sind unter der Adipinsäure, der $\alpha$-Amino-adipinsäure, der Sebacinsäure, der trans-10-Hydroxy-$\Delta^2$-decenoylsäure und der trans-Oxo-9-decen-2-oylsäure .

**5.** Konjugat nach einem der Ansprüche 1 bis 4, **dadurch gekennzeichnet, dass** es unter den folgenden Peptidderivaten ausgewählt ist :

1.  $A - Me.Nle - Glu - His - D\text{-homo-Phe} - Arg - Trp - Gly - NH_2$

2.  $A - Me.Nle - Glu - His - D\text{-homo-Phe} - Arg - Trp - NH_2$

3.  A - Me.Nle – Glu – His - D-homo- Phe – Arg - NH$_2$

4.  A – Me.Nle – Glu – His - D-homo-Phe - NH$_2$

5.  A - Me.Nle – Glu – His - para-fluor-Phe – Arg – Trp – Gly -NH$_2$

6.  A - Me.Nle – Glu – His - para-fluor-Phe – Arg – Trp - NH$_2$

7.  A - Me.Nle – Glu – His - para-fluor-Phe – Arg - NH$_2$

8.  A - Me.Nle – Glu – His - para-fluor-Phe - NH$_2$

9.  A - N-Me.Nle – Glu – His - D-homo-Phe –Arg – Trp–Gly–NH$_2$

10. A - N-Me.Nle – Glu – His - D-homo-Phe –Arg – Trp –NH$_2$

11. A - N-Me.Nle – Glu – His -D-homo-Phe –Arg – NH$_2$

12. A - N-Me.Nle – Glu – His - D-homo-Phe – NH2

13. A - Me.Nle – Glu – His - D-homo-Phe – Arg –Trp –, Gly - OH

14. A - Me.Nle – Glu – His - D-homo-Phe – Arg –Trp – OH

15. A - Me.Nle – Glu – His - D-Homo-Phe – Arg – OH

16. A - N-Me.Nle – Glu – His - D-homo-Phe – Arg –Trp – Gly – OH

17. A - N-Me.Nle – Glu – His - D-homo-Phe – Arg –Trp – OH

18. A - Me.Nle – Glu – His - para-fluor-Phe – Arg –Trp –Gly – OH

19. A - Me.Nle – Glu – His - para-fluor-Phe – Arg –Trp - OH

20. A - Me.Nle – Glu – His - para-fluor-Phe – Arg - OH

21. A -N – Me.Nle – Glu – His - para-fluor-Phe – Arg – Trp -Gly - OH

22. A - N -Me.Nle - Glu - His - para-fluor-Phe - Arg - Trp - OH

worin A eine Säure der allgemeinen Formeln I oder II, wie in den Ansprüchen 1, 3 und 4 definiert, sowie die Derivate dieser Moleküle in Form ihrer Salze, Ester oder Amide ist.

6. Die Konjugate nach einem der Ansprüche 1 bis 5 als Medikament.

7. Medikament nach Anspruch 6 für die Behandlung der Allergien, insbesondere von Hautallergien, entzündlichen Reaktionen und Störungen der Melanogenese, für Anwendungen in menschlicher und tierärtzlicher Medizin.

8. Galenische Zusammensetzung, **dadurch gekennzeichnet, dass** sie ein Konjugat nach einem der Ansprüche 1 bis 5 enthält.

9. Galenische Zusammensetzung nach Anspruch 8, **dadurch gekennzeichnet, dass** es sich um dermato-kosmetische Zubereitungen in Form von Lösungen, Lotionen-Emulsionen oder Cremes handelt, welche als Hautbräunungsbeschleuniger ohne UV Bestrahlung verwendet werden.

10. Galenische Zusammensetzung nach einem der Ansprüche 8 und 9, **dadurch gekennzeichnet, dass** es sich um galenische Zubereitungen in Form von Lösungen, Lotionen, Cremes oder Spray handelt.